(19) Europäisches Patentamt
European Patent Office
Office européen des brevets

(11) Veröffentlichungsnummer: **0 044 412**
**B1**

(12) **EUROPÄISCHE PATENTSCHRIFT**

(45) Veröffentlichungstag der Patentschrift: **25.01.84**

(51) Int. Cl.³: **C 07 C 29/14,** C 07 C 31/20, C 07 C 31/27, C 07 C 33/26

(21) Anmeldenummer: **81104693.7**

(22) Anmeldetag: **19.06.81**

(54) **Verfahren zur Herstellung von Propandiolen.**

(30) Priorität: **23.07.80 DE 3027889**

(43) Veröffentlichungstag der Anmeldung: **27.01.82 Patentblatt 82/4**

(45) Bekanntmachung des Hinweises auf die Patenterteilung: **25.01.84 Patentblatt 84/4**

(84) Benannte Vertragsstaaten: **BE CH DE FR GB LI**

(56) Entgegenhaltungen:
**EP - A - 0 006 460**
**EP - A - 0 008 767**
**DE - A - 2 132 020**
**DE - A - 2 653 096**
**DE - B - 1 905 547**
**US - A - 3 431 311**

(73) Patentinhaber: **BASF Aktiengesellschaft, Carl-Bosch-Strasse 38, D-6700 Ludwigshafen (DE)**

(72) Erfinder: **Broecker, Franz Josef, Dr., Schwanthaler Allee 20, D-6700 Ludwigshafen (DE)**
Erfinder: **Hupfer, Leopold, Dr., Waltershoehe 3, D-6701 Friedelsheim (DE)**
Erfinder: **Merger, Franz, Dr., Max-Slevogt-Strasse 25, D-6710 Frankenthal (DE)**
Erfinder: **Miesen, Ernest, Samariterstrasse 3, D-6700 Ludwigshafen (DE)**
Erfinder: **Paetsch, Juergen, Dr., Am Altenbach 30, D-6706 Wachenheim (DE)**
Erfinder: **Zirker, Guenter, Dr., Schelmenzeile 78, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 044 412 B1

ACTORUM AG

# Verfahren zur Herstellung von Propandiolen

Die Erfindung betrifft die Herstellung von Propandiolen der Formel

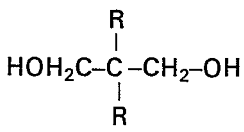
I,

worin die einzelnen Reste R gleich oder verschieden sein können und jeweils einen aliphatischen, araliphatischen oder aromatischen Rest bedeuten, oder beide Reste R zusammen mit dem benachbarten Kohlenstoffatom Glieder eines alicyclischen Ringes bezeichnen, durch Hydrierung von Hydroxypropionaldehyden der Formel

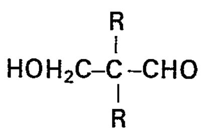
II,

in flüssiger Phase mit Hydrierkatalysatoren, die aus durch Fällung erhaltenen Kupfer-Zink-Mischkristallen der Formel

$Cu_{1,5\ bis\ 3}\ Zn_{1\ bis\ 2,5}\ (CO_3)_{1\ bis\ 2}\ (OH)_{4\ bis\ 6} \cdot (H_2O)_{0\ bis\ 1}$

durch Zersetzung bei 200 bis 500 °C hergestellt werden.

Die Hydrierung von Hydroxypivalinaldehyd erfolgt üblicherweise in flüssiger Phase bei Drücken von 100 bis 200 bar und Temperaturen bis 150 °C unter Verwendung von Nickel-, Nickel-Kupfer- und Kobaltkatalysatoren (DE-AS 10 14 089). Die DE-OS 18 04 984 beschreibt die Hydrierung bei 175 bis 220 °C und 64 bis 704 bar in Gegenwart eines Kupferchromitkatalysators. In dieser Patentschrift wird darauf hingewiesen (Seite 10, letzter Absatz), dass die Wahl des eingesetzten Katalysators von entscheidender Bedeutung ist, da die ydrierung in Gegenwart von Formaldehyd und Wasser erfolgt. Die meisten gängigen Hydrierkatalysatoren werden von Formaldehyd desaktiviert. Ausserdem wird die Aktivität und Stabilität der meisten Trägermaterialien durch Wasser nachteilig beeinflusst. Mit Nickel- und Kobaltkatalysatoren werden unter den angegebenen Bedingungen unerwünschte Nebenprodukte gebildet, wodurch die Ausbeute und Reinheit des Neopentylglykols beeinträchtigt wird.

Bei einem weiteren Verfahren (britische Patentschrift 1 048 530) wird 2,2-Dimethyl-3-hydroxypropanal gleichzeitig mit Isobutyraldehyd in Gegenwart eines Kupfer-Chromoxid-Katalysators hydriert. Auch bei diesem Verfahren treten zahlreiche Nebenprodukte in deutlichem Masse auf. Wirtschaftlich einschränkend ist der Zwang, die anfallenden Mengen Isobutanol weiterzuverarbeiten.

Um befriedigende Ausbeuten zu erzielen, müssen extreme Hydrierbedingungen angewandt und aufwendige Rückführungen durchgeführt werden. Ebenso aufwendig ist gegebenenfalls die Aufarbeitung bzw. die mit erheblichem Materialverlust verbundene Entsorgung der wässrigen Phase.

Es ist aus der deutschen Auslegeschrift DE-B 19 57 551 bekannt, dass man Neopentylglykol durch Umsetzung von Isobutyraldehyd und Formaldehyd in Gegenwart eines basischen Katalysators und anschliessender Hydrierung des gebildeten 2,2-Dimethyl-3-hydroxypropanals in Gegenwart eines Hydrierkatalysators herstellt. Als Hydrierkatalysatoren werden Kobalt-, Kupfer-, Mangan- und/oder Nickelkatalysatoren, z.B. entsprechende Sinterkatalysatoren, genannt. Bevorzugt wird als Zusatzkomponente Phosphorsäure verwendet. In den Beispielen werden hohe Hydriertemperaturen angewandt, wodurch erst befriedigende Raum-Zeit-Ausbeuten erzielt werden.

Es wurde nun gefunden, dass man Propandiole der Formel

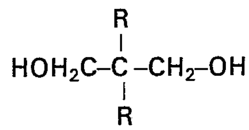
I,

worin die einzelnen Reste R gleich oder verschieden sein können und jeweils einen aliphatischen, araliphatischen oder aromatischen Rest bedeuten, oder beide Reste R zusammen mit dem benachbarten Kohlenstoffatom Glieder eines alicyclischen Ringes bezeichnen, durch Hydrierung von Hydroxypropionaldehyden der Formel

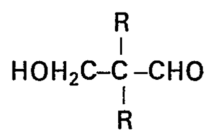
II,

worin R die vorgenannte Bedeutung besitzt, in Gegenwart eines Kupfer enthaltenden Hydrierkatalysators vorteilhaft erhält, wenn man die Hydrierung in flüssiger Phase mit Hydrierkatalysatoren durchführt, die dadurch erhalten worden sind, dass man Kupfer und Zink in einem Verhältnis von 0,6 bis 3 Atomen Kupfer zu einem Atom Zink aus ihren Verbindungen in Gegenwart von Carbonaten bei einem pH von 6,9 bis 8 fällt und die so erhaltenen Mischkristalle der Formel

$Cu_{1,5\ bis\ 3}\ Zn_{1\ bis\ 2,5}\ (CO_3)_{1\ bis\ 2}\ (OH)_{4\ bis\ 6} \cdot (H_2O)_{0\ bis\ 1}$

bei einer Temperatur von 200 bis 500 °C zersetzt.

Die Umsetzung lässt sich für den Fall der Verwendung von Hydroxypivalinaldehyd durch die folgenden Formeln wiedergeben:

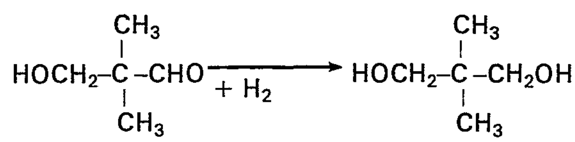

Im Hinblick auf die bekannten Verfahren liefert das Verfahren nach der Erfindung Propandiole, insbesondere Neopentylglykol auf einfacherem und wirtschaftlicherem Wege in guter Ausbeute und Reinheit ohne wesentliche Bildung von Nebenprodukten, z.B. Estern und Acetalen, und Zersetzungsprodukten. Zusätzliche Reinigungsoperationen oder Zusatzstoffe, die vor der Hydrierung entfernt werden müssen, sind nicht notwendig. Die hohe Hydrieraktivität des erfindungsgemässen Katalysators ermöglicht die Hydrierung bei niedrigen Temperaturen durchzuführen, so dass z.B. eine hydrogenolytische Spaltung des gebildeten Neopentylglykols nicht auftritt. Auch die Rückspaltung von Hydroxypivalinaldehyd in Isobutyraldehyd und Formaldehyd wird nicht beobachtet. Alle diese vorteilhaften Ergebnisse sind überraschend, insbesondere die geringe Esterbildung, denn Zinkoxid ist als guter Katalysator für die Bildung von Hydroxypivalinsäureneopentylglykolester bekannt (DE-PS 22 34 110). Das erfindungsgemässe Verfahren bietet im Hinblick auf DE-AS 19 57 591 den Vorteil einer Hydrierung des rohen unbehandelten Hydroxypivalinaldehyds bei niedrigen Temperaturen und Drücken in flüssiger Phase mit hoher Raum-Zeit-Ausbeute. Der Katalysator zeichnet sich durch hohe Aktivität, lange Lebensdauer und mechanische Stabilität aus.

Ein wesentliches Merkmal des erfindungsgemässen Verfahrens besteht darin, dass der Hydrierkatalysator aus speziell strukturierten Mischkristallen hergestellt wird; diese Struktur hält bei der anschliessenden thermischen Zersetzung (Calcinierung) die Feinverteilung und eine besondere Konfiguration von Kupfer und Zink im fertigen, d.h. calcinierten Katalysator aufrecht. Diese Konfiguration liefert die vorteilhaften erfindungsgemässen Ergebnisse. Die durch Fällung gebildeten Kupfer/Zink/$CO_3$/OH-Mischkristalle haben ein definiertes und messbares Kristallgitter. Bevorzugt liegen die Kristalle in einem monoklinen Kristallsystem vor. Die einzelnen Gitterplätze werden von Kupfer und Zink sowie den Resten $CO_3$ und OH eingenommen, wobei noch Zusatzstoffe, in der Regel in einer Menge von 0 bis 10, insbesondere 0 bis 5 Gewichtsprozent, bezogen auf den Gesamtkatalysator, Gitterplätze belegen können. Als Zusatzstoffe kommen zweckmässig Chrom, Calzium, Magnesium und bevorzugt Aluminium in Betracht, die in Gestalt ihrer Carbonate, Hydroxide oder Oxide als Gitterpunkte eingebaut werden oder Fehlstellen besetzen; gegebenenfalls können die Zusatzstoffe auch in amorpher oder kristalliner Form im Gemenge mit den erfindungsgemässen Mischkristallen zur Calcinierung gelangen. In den Mischkristallen stehen die erfindungsgemässen Komponenten in einem Verhältnis von 1,5 bis 3, insbesondere 1,75 bis 2,5 Atom Kupfer zu 1 bis 2,5, insbesondere 1,5 bis 2,25 Atomen Zink, zu 1 bis 2 Äquivalenten $CO_3$ und zu 4 bis 6 Äquivalenten OH. Bevorzugt sind Mischkristalle, die in ihrem Gitteraufbau dem Hydrozinkits und insbesondere dem Malachit und Zinkhydroxycarbonat entsprechen.

Die Erfindung geht von der Beobachtung aus, dass bei der Fällung von Kupfer- und Zinkhydroxycarbonaten je nach pH-Bedingungen und Atomverhältnissen von Kupfer und Zink Mischkristalle auftreten. Überwiegt Kupfer in der zu fällenden Lösung, so entsteht ein Mischkristall, der eines der vorgenannten Kristallgitter, z.B. das Kristallgitter des Malachits, besitzt, während andererseits bei einem überwiegenden Zinkgehalt ein Mischkristall, der eines der vorgenannten Kristallgitter, z.B. das Kristallgitter eines Zinkhydroxidcarbonats, besitzt, entsteht. So kann im Malachit $CuCO_3 \cdot Cu(OH)_2$ ein Teil der Kupferionen durch Zinkionen und zum anderen in einem Zinkhydroxidcarbonat, z.B. $ZnCO_3 \cdot 3\,Zn(OH)_2 \cdot H_2O$ ein Teil der Zinkionen durch Kupferionen ersetzt sein. Durch die Herstellung dieser Mischkristallverbindungen werden die Aktivkomponenten optimal verteilt. Diese Verteilung überträgt sich bei der Zersetzung der Hydroxidcarbonate während der Kalzination auf das Oxidgemisch.

Bevorzugte Ausgangsstoffe II und demzufolge bevorzugte Endstoffe I sind solche, in deren Formeln die einzelnen Reste R gleich oder verschieden sind und jeweils einen Alkylrest mit 1 bis 6 Kohlenstoffatomen, einen Aralkylrest oder Alkylarylrest mit jeweils 7 bis 12 Kohlenstoffatomen, oder einen Phenylrest bedeuten, oder beide Reste R zusammen mit dem benachbarten Kohlenstoffatom Glieder eines 5- oder 6-gliedrigen alicyclischen Ringes bezeichnen. Die vorgenannten Reste und Ringe können noch durch unter den Reaktionsbedingungen inerte Gruppen, z.B. Alkylgruppen oder Alkoxygruppen mit jeweils 1 bis 4 Kohlenstoffatomen, substituiert sein.

Als Ausgangsstoffe II kommen z.B. in Frage: In 2-Stellung gleich oder unterschiedlich zweifach durch Methyl-, Äthyl-, Propyl-, Isopropyl-, Butyl-, Isobutyl-, sek.-Butyl-, tert.-Butyl-, Benzyl-, Phenyl-, 2-Methylphenyl-, 3-Methylphenyl-, 4-Methylphenyl-, 3-Methoxyphenyl-, 2-Methoxyphenyl-, 4-Methoxyphenyl-gruppen substituierte 3-Hydroxypropionaldehyde; 1-Formyl-1-methylolcyclohexan, 1-Formyl-1-methylolcyclopentan.

Die Fällung erfolgt zweckmässig durch Vermischen der Lösungen von Verbindungen des Kupfers, Zinks und der Zusatzstoffe, vorzugsweise des Aluminiums. Zwar können die Zusatzstoffe auch nachträglich in das Fällbad gegeben oder mit den Mischkristallen vor der Calcinierung bzw. der Katalysatorteilchen nach der Calcinierung vermischt werden, in der Regel wird man sie aber in Gestalt von Lösungen zusammen mit Kupfer und Zink bei der Fällung ausfällen. Damit während der Kalzination eine möglichst geringe Rekristallisation erfolgt, ist es vorteilhaft, solche Zusatzstoffe als strukturelle Promotoren bzw. Stabilisatoren, beispielsweise Aluminiumoxid oder Chromoxid, zusammen mit den Mischkristallen auszufällen. Die Fällung wird zweckmässig aus wässrigen Lösungen und mit entsprechend wasserlöslichen Verbindungen der Metalle durchgeführt. Als Verbindungen kommen z.B. die Acetate, Formiate, Chloride, Bromide, Jodide, Sulfate, Hydroxide, Carbonate, Bicarbonate, Hydrogensulfate, Phosphate, Hydrogenphosphate, Dihydrogenphosphate, Nitrite und insbesondere Nitrate von Kupfer, Zink und der Zusatzmetalle in Betracht. Die Carbonatreste im Mischkristall können in das Fällungsbad in Gestalt entsprechender Carbonate von Kupfer, Zink oder Zusatzmetall, oder zweckmässig in Gestalt von zusätzlichen, den erfindungsgemässen pH-Wert einstellenden Alkalicarbonaten oder Alkalibicarbonaten, z.B. den Kalium- oder Natriumsalzen, zugegeben werden. Der erfindungsgemäss verwendete Mischkristall wird vorteilhaft mittels zweier wässriger Lösungen hergestellt, wobei Lösung 1 die Nitrate von Kupfer, Zink und Zusatzmetall, z.B. Aluminium, im oben angegebenen Atomverhältnis enthält. Die Lösung 2 besteht aus einer wässrigen Natriumcarbonatlösung. Zweckmässig verwendet man 1- bis 2-molare Lösungen der Metallverbindungen und 1- bis 2-molare Carbonatlösungen bzw. Bicarbonatlösungen. Die Fällung wird vorteilhaft bei einer Temperatur von 5 bis 90, bevorzugt 20 bis 90, zweckmässig 40 bis 85, insbesondere 60 bis 80 °C, während 1 bis 2 Stunden, diskontinuierlich oder kontinuierlich, drucklos oder unter Druck, bei einem pH von 7 bis 8, insbesondere 7 bis 7,5, durchgeführt. Bevorzugt werden beide Lösungen auf die Fällungstemperatur erhitzt und in einen Rührkessel geleitet. Durch Regelung der Zulaufgeschwindigkeiten wird bei dieser Parallelfällung der vorgenannte pH-Wert eingehalten. Der erfindungsgemässe pH-Wert muss von Anfang an während der gesamten Fällungszeit beibehalten werden. Der entstandene Niederschlag wird dann zweckmässig 15 bis 60 Minuten bei pH 7 bis 7,5, insbesondere pH 7, nachgerührt, abfiltriert und mit Wasser salzfrei, z.B. nitratfrei, gewaschen. Die gebildeten Mischkristalle in der Fällung können schnell röntgenographisch durch Debye-Scherrer-Aufnahmen geprüft werden. Der gewaschene Niederschlag wird dann zweckmässig bei 100 bis 200 °C getrocknet und anschliessend zweckmässig bei 250 bis 500 °C, vorzugsweise 300 bis 400 °C, vorteilhaft während 2 bis 5 Stunden, kalziniert. Danach wird vorteilhaft der oxidische Katalysator in die gewünschte Form gebracht, z.B. zu Pillen oder Strängen verformt. Nach der Verformung wird der Katalysator zweckmässigerweise nochmals auf 200 bis 500, insbesondere 300 bis 500 °C erhitzt. Der so hergestellte Katalysator wird dann in den Hydrierreaktor eingegeben. In einer bevorzugten Ausführungsform wird er dann bei einer Temperatur von 150 bis 250 °C mit einem Gemisch aus 0,5 bis 30 Volumenteilen Wasserstoff und 99,5 bis 70 Volumenteilen Stickstoff aktiviert. Nach der Aktivierung wird der Katalysator zweckmässig mit Wasser oder dem Propandiol enthaltenden Hydriergemisch benetzt.

Die Hydrierung der Hydroxypropionaldehyde II wird zweckmässig diskontinuierlich oder kontinuierlich bei Temperaturen zwischen 80 und 260 °C, vorzugsweise 100 bis 175 °C, insbesondere 110 bis 150 °C, und Drücken von 1 bis 300 bar, vorzugsweise 10 bis 200 bar, insbesondere 20 bis 100 bar, durchgeführt.

Bei diskontinuierlichen Hydrierungen verwendet man den Hydrierkatalysator in der Regel in einer Menge von 1 bis 10, insbesondere 2 bis 5 Gewichtsprozent, bezogen auf Hydroxypivalinaldehyd, und Reaktionszeiten von 0,1 bis 5 Stunden.

Die kontinuierliche Hydrierung erfolgt zweckmässig nach konventionellen Techniken, z.B. in Sumpf- oder Rieselfahrweise in einem Festbettreaktor bei den genannten Temperaturen und Drükken. Man führt Hydroxypivalinaldehyd II unter den Vorzugsbedingungen zweckmässig mit Hilfe von Einspritzpumpen kontinuierlich in Mengen von 0,1 bis 5 Teilen je Liter Katalysator und Stunde in den Reaktor ein. Der Reaktor-Austrag wird entweder unmittelbar in eine kontinuierlich arbeitende Fraktionierkolonne eingeführt oder in Vorratsbehältern gesammelt und chargenweise fraktioniert. Man wählt im allgemeinen den Destillationsdruck so, dass der Siedepunkt des Endstoffes höher liegt als sein Schmelzpunkt (Fp 129 °C), d.h. oberhalb 50 mbar.

In einer bevorzugten Ausführungsform verwendet man anstelle der Ausgangsstoffe II das Reaktionsgemisch der Herstellung von Ausgangsstoff II, z.B. anstelle von Hydroxypivalinaldehyd das Reaktionsgemisch der Umsetzung von Isobutyraldehyd und Formaldehyd, zweckmässig in einer Menge von 0,1 bis 1,5, vorteilhaft 0,9 bis 1,1 Mol Formaldehyd je Mol Isobutyraldehyd und in Gegenwart von tertiären Aminen als Katalysatoren, vorteilhaft bei einer Temperatur von 20 bis 100 °C, drucklos oder unter Druck, diskontinuierlich, z.B. während 0,1 bis 4 Stunden, oder kontinuierlich.

Die nach dem Verfahren der Erfindung herstellbaren Propandiole-(1,3) sind wertvolle Ausgangsstoffe für die Herstellung von Schmiermitteln, Kunststoffen, Lacken und Kunstharzen, z.B. entsprechenden Polyestern. Bezüglich der Verwendung wird auf die genannten Veröffentlichungen und Ullmanns Encyklopädie der technischen Chemie, Band 15, Seiten 292 ff, verwiesen.

Die in den Beispielen angegebenen Teile bedeuten Gewichtsteile. Sie verhalten sich zu den Volumenteilen wie Kilogramm zu Liter.

Beispiel 1

a) Herstellung des Katalysators: Der Katalysator wird aus einem Aluminium enthaltenden Mischkristall vom Zinkhydroxidcarbonattyp (4 $ZnO \cdot CO_2 \cdot 4\ H_2O$) hergestellt. Die mit Aluminiumoxid dotierte Verbindung mit folgender Zusammensetzung der Mischkristalle

$$Cu_{1,76}Zn_{2,24}(CO_3)(OH)_6 \cdot H_2O$$

wird aus zwei Lösungen hergestellt:

Lösung 1:

7,200 Teile $Cu(NO_3)_2 \cdot 3\ H_2O$

11,370 Teile $Zn(NO_3)_2 \cdot 6\ H_2O$ und 1,473 Teile $Al(NO_3)_2 \cdot 9\ H_2O$ werden in Wasser gelöst. Die

Lösung wird mit Wasser zu 36 Volumenteilen aufgefüllt.

Lösung 2:

Mit 7,850 Teilen Soda werden 37 Volumenteile wässrige Lösung hergestellt.

Beide Lösungen werden getrennt auf 80 °C erhitzt. Die Fällung erfolgt dann in einem Rührkessel, in dem 10 Volumenteile Wasser von 80 °C vorgelegt werden. Unter kräftigem Rühren werden durch parallelen Zulauf von Lösung 1 und 2 die Mischkristalle gefällt. Durch genaue Einstellung der Zulaufgeschwindigkeiten wird während der gesamten Fällungsperiode (ca. 1 Stunde) ein pH-Wert von 7 im Rührkessel aufrechterhalten. Die pH-Wert-Kontrolle erfolgt mit einer Glaselektrode.

Nach Beendigung der Fällung wird das Gemisch noch 30 Minuten bei 80 °C nachgerührt und dann der Niederschlag filtriert und nitratfrei gewaschen. Eine Probe der Mischkristallfällung wird mittels einer Guinier-Aufnahme röntgenographisch untersucht. Folgende d-Werte der Beugungslinien charakterisieren die gebildete Mischkristallverbindung:

6,76; 3,71; 3,20; 2,89; 2,75; 2,63; 2,16; 1,95; 1,60 Der Filterkuchen wird bei 110 °C getrocknet und anschliessend 5 Stunden bei 300 °C kalziniert. Das entstandene oxidische Produkt wird auf eine Korngrösse unter 1 mm zerkleinert und nach Zusatz von 2 Gewichtsprozent Graphit zu Pillen verpresst.

b) Hydrierung: 2 Teile des nach a) dargestellten Katalysators werden in Form von 5 × 5 mm-Pillen in einen Hydrierreaktor eingebracht und mit 500 Volumenteilen eines Gemischs aus 5 Volumenprozent $H_2$ und 95 Volumenprozent $N_2$ bei einer Temperatur von 180 °C drucklos reduziert. Nach der Aktivierung wird der Katalysator auf 100 °C abgekühlt und dann aus einem Behälter stündlich ein Teil einer 60-gewichtsprozentigen, wässrigen Lösung von Hydroxypivalinaldehyd (Reinheit 98%; Nebenkomponenten Isobutyraldehyd und Formaldehyd) durch den Reaktor geführt und bei 130 °C und 30 bar hydriert. Die Belastung beträgt 0,3 Teile Hydroxypivalinaldehyd je Liter Katalysator und Stunde. Der Umsatz beträgt 99 Prozent. Der Hydrieraustrag wird vom Katalysator abfiltriert und über eine Glasfüllkörperkolonne fraktioniert. Man erhält bei 90 mbar nach Vorläufen aus Wasser und geringen Mengen Methanol, Isobutanol und Hydroxypivalinaldehyd Neopentylglykol vom Siedepunkt 138 bis 140 °C (Schmelzpunkt 128 bis 129 °C. Der Estergehalt liegt unter 0,1 Gewichtsprozent), entsprechend 0,5965 Teilen je Stunde. Dies entspricht einer Ausbeute von 97,5% der Theorie, bezogen auf den Ausgangsstoff.

Beispiel 2

a) Herstellung des Katalysators: Zur Herstellung eines kupferreichen Katalysators wird ein Aluminium enthaltender Mischkristall vom Malachittyp mit folgender Summenformel hergestellt:

$Cu_{2,4}Zn_{1,6}(CO_3)_2\,(OH)_4$

Zur Fällung verwendet man wie in Beispiel 1a) zwei Lösungen.

Lösung 1:

14,790 Teile $Cu(NO_3)_2 \cdot 3\,H_2O$

11,540 Teile $Zn(NO_3)_2 \cdot 6\,H_2O$

2,240 Teile $Al(NO_3)_3 \cdot 9\,H_2O$

werden in Wasser gelöst. Die Lösung wird zu 32 Volumenteilen Lösung aufgefüllt.

Lösung 2:

In 32 Volumenteilen Wasser werden 8,060 Teile Soda gelöst.

Beide Lösungen werden auf 80 °C erhitzt und in einem Rührkessel mit 8 Volumenteilen Wasser als Vorlage bei 80 °C und einem pH-Wert von 7 durch parallelen geregelten Zulauf in 45 Minuten vereinigt. Der erhaltene Niederschlag wird abfiltriert, nitratfrei gewaschen und bei 110 °C getrocknet.

Das getrocknete Produkt besteht nach einem Debye-Scherrer-Diagramm aus einer einheitlichen Verbindung mit folgenden d-Werten der Röntgenbeugungslinien in der Reihenfolge ihrer Intensitäten:

3,70; 2,72; 5,10; 6,10; 2,45; 2,55; 7,60
3,00; 6,90; 2,68; 2,32; 2,18; 1,64; 1,52

Nach dem Trocknen werden die Mischkristalle 7 Stunden bei 300 °C kalziniert und dann nach Zusatz von 2 Gewichtsprozent Graphit zu Pillen verpresst. Die Pillen werden dann nochmals 3 Stunden bei 300 °C nachkalziniert.

b) Hydrierung: Analog Beispiel 1b) wird die Hydrierung bei 110 °C durchgeführt und das Reaktionsgemisch aufgearbeitet. Die Belastung beträgt:

2b 1) 0,3 Teile Hydroxypivalinaldehyd je Liter Katalysator und Stunde (Umsatz 98%). Durch fraktionierende Destillation erhält man Neopentylglykol vom Schmelzpunkt 129 °C, in einer Menge entsprechend 0,5934 Teilen je Stunde. Dies entspricht einer Ausbeute von 97% der Theorie, bezogen auf den Ausgangsstoff.

2b 2) Analog Beispiel 1b) wird die Hydrierung bei 130 °C durchgeführt. Man erzielt 99% Umsatz und erhält durch fraktionierende Destillation Neopentylglykol vom Schmelzpunkt 129 °C, in einer Menge entsprechend 0,5995 Teilen je Stunde. Dies entspricht einer Ausbeute von 98% der Theorie, bezogen auf den Ausgangsstoff.

2b 3) Analog Beispiel 2b 2) werden 0,6 Teile Hydroxypivalinaldehyd je Liter Katalysator und Stunde hydriert. Man erzielt 87% Umsatz und erhält durch fraktionierende Destillation nach Abtrennung eines Vorlaufs und rückführbarem Ausgangsstoff Neopentylglykol vom Schmelzpunkt 128 bis 129 °C, in einer Menge entsprechend 1,0474 Teilen je Stunde. Dies entspricht einer Selektivität von 98,4%, bezogen auf umgesetzten Ausgangsstoff.

Beispiel 3

Der im Beispiel 1a) beschriebene Katalysator

wird 1 000 Stunden bei 110 °C, einem Druck von 30 bar und einer Belastung von 0,3 Teile Hydroxypivalinaldehyd je Liter Katalysator und Stunde in einem Kreislauf-Hydrierreaktor verwendet.

Hydrierung und Aufarbeitung erfolgen analog Beispiel 1b). Der Umsatz beträgt 98 Prozent. Dieser Umsatz bleibt über 1 000 Betriebsstunden ohne Aktivitätsverlust des Katalysators konstant.

Durch fraktionierende Destillation erhält man Neopentylglykol vom Schmelzpunkt 128 bis 129 °C, in Mengen die während der gesamten Betriebszeit 0,5933 Teilen je Liter Katalysator und Stunde entsprechen. Dies entspricht einer Ausbeute von 97%, bezogen auf den Ausgangsstoff.

Beispiel 4

Entsprechend Beispiel 1b wird stündlich ein Teil einer 70-gewichtsprozentigen, wässrigen Lösung von 2-Methyl-2-äthyl-3-hydroxypropanal (an dem nach Beispiel 1a hergestellten Katalysator) bei 130 °C, 30 bar und einer Belastung von 0,35 Teilen 2-Methyl-2-äthyl-3-hydroxypropanal je Liter Katalysator und Stunde hydriert. Der Umsatz beträgt 99 Prozent. Der Hydrieraustrag wird über eine Füllkörperkolonne fraktioniert. Man erhält nach Vorläufen aus Wasser und geringen Mengen Methanol 2-Methylbutanol und 2-Methyl-2-äthyl-3-hydroxypropanal bei 12 mbar 2-Methyl-2-äthylpropandiol-(1,3) vom Siedepunkt 110 bis 112 °C (Schmelzpunkt 44 bis 46 °C), entsprechend 0,6850 Teilen je Stunde. Dies entspricht einer Ausbeute von 96,2% der Theorie, bezogen auf den Ausgangsstoff.

Beispiel 5

Entsprechend Beispiel 1b werden stündlich 1,4 Teile einer 60-gewichtsprozentigen Lösung von 1-Hydroxymethyl-hexahydro-benzaldehyd in Wasser-Methanol (1:1) (an dem nach Beispiel 1a hergestellten Katalysator) bei 130 °C, 30 bar und einer Belastung von 0,42 Teilen 1-Hydroxymethyl-hexahydrobenzaldehyd je Liter Katalysator und Stunde hydriert. Der Umsatz beträgt 99 Prozent. Der Hydrieraustrag wird über eine Füllkörperkolonne fraktiniert. Man erhält nach Vorläufen aus Wasser, Methanol und geringen Mengen Hexahydrobenzylalkohol und 1-Hydroxymethyl-hexahydrobenzaldehyd bei 3 mbar 1,1-Di-hydroxymethyl-cyclohexan vom Siedepunkt 120 bis 122 °C (Schmelzpunkt 90 bis 92 °C), entsprechend 0,8314 Teilen je Stunde. Dies entspricht einer Ausbeute von 97,6% der Theorie, bezogen auf den Ausgangsstoff.

Beispiel 6

Entsprechend Beispiel 1b wird stündlich ein Teil einer 72-gewichtsprozentigen, wässrigen Lösung von 2-Methyl-2-propyl-3-hydroxypropanal (an dem nach Beispiel 1a hergestellten Katalysator) bei 130 °C, 30 bar und einer Belastung von 0,36 Teilen 2-Methyl-2-propyl-3-hydroxypropanal je Liter Katalysator und Stunde hydriert. Der Umsatz beträgt 80,5 Prozent. Der Hydrieraustrag wird über eine Füllkörperkolonne fraktioniert. Man erhält nach Vorläufen aus Wasser und geringen Mengen Methanol 2-Methylpentanol und 2-Methyl-2-propyl-3-hydroxypropanal bei 5 mbar 2-Methyl-2-propylpropandiol-(1,3) vom Siedepunkt 112 bis 113 °C (Schmelzpunkt 58 bis 60 °C), entsprechend 0,6967 Teilen je Stunde. Dies entspricht einer Ausbeute von 95,3% der Theorie, bezogen auf den Ausgangsstoff.

Beispiel 7

Entsprechend Beispiel 1b werden stündlich 1,2 Teile einer 50-gewichtsprozentigen Lösung von 2-Methyl-2-phenyl-3-hydroxypropanal in Wasser-Methanol (1:1) (an dem nach Beispiel 1a hergestellten Katalysator) bei 130 °C, 30 bar und einer Belastung von 0,3 Teilen 2-Methyl-2-phenyl-3-hydroxypropanal je Liter Katalysator und Stunde hydriert. Der Umsatz beträgt über 99 Prozent. Der Hydrieraustrag wird über eine Füllkörperkolonne fraktioniert. Man erhält nach Vorläufen aus Wasser, Methanol, 2-Phenylpropanol und 2-Methyl-2-phenyl-3-hydroxypropanal bei 1 mbar 2-Methyl-2-phenyl-propandiol-(1,3) vom Siedepunkt 143 bis 145 °C (Schmelzpunkt 80 bis 82 °C), entsprechend 0,5405 Teilen je Stunde. Dies entspricht einer Ausbeute von 89% der Theorie, bezogen auf den Ausgangsstoff.

## Patentanspruch

1. Verfahren zur Herstellung von Propandiolen der Formel

$$HOH_2C-\overset{\overset{\displaystyle R}{|}}{\underset{\underset{\displaystyle R}{|}}{C}}-CH_2-OH \qquad I,$$

worin die einzelnen Reste R gleich oder verschieden sein können und jeweils einen aliphatischen, araliphatischen oder aromatischen Rest bedeuten, oder beide Reste R zusammen mit dem benachbarten Kohlenstoffatom Glieder eines alicyclischen Ringes bezeichnen, durch Hydrierung von Hydroxypropionaldehyden der Formel

$$HOH_2C-\overset{\overset{\displaystyle R}{|}}{\underset{\underset{\displaystyle R}{|}}{C}}-CHO \qquad II,$$

worin R die vorgenannte Bedeutung besitzt, in Gegenwart eines Kupfer enthaltenden Hydrierkatalysators, dadurch gekennzeichnet, dass man die Hydrierung in flüssiger Phase mit Hydrierkatalysatoren durchführt, die dadurch erhalten worden sind, dass man Kupfer zu einem Atom Zink aus ihren Verbindungen in Gegenwart von Carbonaten bei einem pH von 6,9 bis 8 fällt und die so erhaltenen Mischkristalle der Formel

$$Cu_{1,5\ \text{bis}\ 3}\,Zn_{1\ \text{bis}\ 2,5}\,(CO_3)_{1\ \text{bis}\ 2}\,(OH)_{4\ \text{bis}\ 6}\cdot(H_2O)_{0\ \text{bis}\ 1}$$

bei einer Temperatur von 200 bis 500 °C zersetzt.

**Revendication**

Procédé pour la préparation de propane-diols de formule

$$HOH_2C-\overset{\overset{\displaystyle R}{|}}{\underset{\underset{\displaystyle R}{|}}{C}}-CH_2-OH \qquad (I)$$

dans laquelle les différents radicaux R peuvent être semblables ou différents et représentent chacun un radical aliphatique, araliphatique ou aromatique, ou bien les deux radicaux forment, avec l'atome de carbone voisin, des termes d'un noyau alicyclique, par hydrogénation d'aldéhydes hydroxypropioniques de formule

$$HOH_2C-\overset{\overset{\displaystyle R}{|}}{\underset{\underset{\displaystyle R}{|}}{C}}-CHO \qquad (II)$$

dans laquelle R a la signification donnée ci-dessus, en présence d'un catalysator d'hydrogénation contenant du cuivre, caractérisé en ce qu'on effectue l'hydrogénation en phase liquide avec des catalyseurs d'hydrogénation que l'on a obtenus en précipitant du cuivre et du zinc dans un rapport de 0,6 à 3 atomes de cuivre pour 1 atome de zinc, à partir de leurs composés, en présence de carbonates à un pH de 6,9 à 8, et en décomposant à une température de 200 à 500 °C les cristaux mixtes ainsi obtenus, de formule

$$Cu_{1,5\text{ à }3}Zn_{1\text{ à }2,5}(CO_3)_{1\text{ à }2}(OH)_{4\text{ à }6}\cdot(H_2O)_{0\text{ à }1}$$

**Claim**

A process for the preparation of the formula

$$HOH_2C-\overset{\overset{\displaystyle R}{|}}{\underset{\underset{\displaystyle R}{|}}{C}}-CH_2-OH \qquad I,$$

where the R's may be identical or different and each is an aliphatic, araliphatic or aromatic radical, or the two R's together with the adjacent carbon atom are members of an alicyclic ring, by hydrogenating a hydroxypropionaldehyde of the formula

$$HOH_2C-\overset{\overset{\displaystyle R}{|}}{\underset{\underset{\displaystyle R}{|}}{C}}-CHO \qquad II,$$

where R has the above meanings, in the presence of a copper-containing hydrogenation catalyst, wherein the hydrogenation is carried out in the liquid phase with a hydrogenation catalyst which has been obtained by precipitating copper and zinc in a ratio of from 0.6 to 3 atoms of copper per atom of zinc from their compounds in the presence of a carbonate at a pH of from 6.9 to 8, and decomposing the resulting mixed crystals of the formula

$$Cu_{1.5-3}Zn_{1-2.5}(CO_3)_{1-2}(OH)_{4-6}\cdot(H_2O)_{0-1}$$

at from 200 to 500 °C.